# EUROPEAN PATENT APPLICATION

(11) **EP 2 455 900 A1**
(43) Date of publication of application: **23.05.2012**
(21) Application number: 11190112.0
(22) Date of filing: 22.11.2011
(51) Int. Cl.: G06Q 10/06, G06F 19/00

(54) **Method and system for lease management of ultrasonic system**

(30) Priority: 23.11.2010 KR 20100116580
(71) Applicant: Samsung Medison Co., Ltd., Nam-myun Hongchun-gun Kangwon do 250-875 (KR)
(72) Inventor: Nam, Sang Gyu, Seoul (KR)
(74) Representative: Schmid, Wolfgang

(57) **Abstract**

A system for lease management of an ultrasonic system includes a usage information collection unit to collect usage information regarding a usage of the ultrasonic system, and a lease management unit to determine whether the usage information provided from the usage information collection module exceeds a predetermined allowable usage set by a prepaid balance of a user Operation of the ultrasonic system is stopped while displaying a settlement-request screen on a display unit of the ultrasonic system, if the usage information exceeds the allowable usage. A lease server remotely manages a lease of the ultrasonic system through communication with the lease management unit via a network.

## Description

### TECHNICAL FIELD

The present disclosure relates to a method and system for managing lease of an ultrasonic system, by which a user can use the ultrasonic system without an excessive burden of initial costs and a provider of the ultrasonic system can efficiently lease out the ultrasonic system.

### BACKGROUND

Ultrasonic diagnostic apparatuses are used in a wide range of applications. For example, ultrasonic diagnostic apparatuses are extensively used in the medical field due to non-invasive and non-destructive characteristics f to human bodies. Recently, high-performance ultrasonic diagnostic apparatuses have been used to provide two-dimensional or three-dimensional images of internal organs.

However, since such an ultrasonic system, while widely used in the medical field, is very expensive, a user who wants to use such system must bear heavy initial costs. Thus, relatively small hospitals have difficulty in purchasing ultrasonic systems due to the cost thereof.

### SUMMARY

To improve over the art and address one or more of the needs outlined above, the present disclosure provides a method and system for leasing an ultrasonic system, by which a user can use the ultrasonic system without an excessive burden of initial costs and a provider of the ultrasonic system can efficiently lease out the ultrasonic system.

An aspect of the disclosure encompasses a lease management system for an ultrasonic system. The ultrasonic system includes: a usage information collection unit to collect usage information regarding a usage of the ultrasonic system and a lease management unit to determine whether the usage information provided from the usage information collection unit exceeds a predetermined allowable usage set by a prepaid lease balance of a user. The lease management module stops operation of the ultrasonic system while displaying a settlement-request screen on a display unit of the ultrasonic system, if the usage information exceeds the allowable usage..A lease server remotely manages a lease of the ultrasonic system through communication with the lease management unit via a network.

The system may further include a settlement unit communicating with the lease server to carry out a settlement process. The lease management unit requests the lease server to process a settlement according to settlement information input through the settlement-request screen by a user. The lease server requests the settlement unit to process the settlement, and the settlement unit carries out the settlement process and a charging process according to the settlement request.

Another aspect of the disclosure relates to a lease management method for an ultrasonic system. Usage information regarding a usage of an ultrasonic system is collected by a usage information collection unit of the ultrasonic system during operation of the ultrasonic system. A lease management module of the ultrasonic system determines whether the usage information exceeds a predetermined allowable usage set by a prepaid lease balance of a user. If the usage information exceeds the predetermined allowable usage, the operation of the ultrasonic system is stopped while displaying a settlement-request screen on a display unit of the ultrasonic system.

The method may further include receiving settlement information from a user through the settlement-request screen. The lease management, unit requests a lease server to process a settlement via a network according to the settlement information inputted by the user. The lease server requests a settlement unit to process the settlement request, and the settlement unit carries out a settlement process and a charging process according to the settlement request.

The method may further include providing a settlement process result from the settlement unit to the lease server and providing information about the settlement process result from the lease server to the lease management unit. The lease management module updates the ultrasonic system into an operable mode in response to the information on the settlement process result.

The usage information may include at least one of duration and frequency of use of the ultrasonic system.

The duration and frequency of use of the ultrasonic system may be linked with use of at least one of a main body, a probe, and an optional or peripheral device of the ultrasonic system.

The method and system for leasing an ultrasonic system according to exemplary embodiments may permit a user to use the ultrasonic system without an excessive burden of initial cost, and permit a provider of the ultrasonic system to efficiently lease out the ultrasonic system.

### BRIEF DESCRIPTION OF THE DRAWINGS

The figures depict one or more implementations in accordance with the present teachings, by way of example only, not by way of limitation. In the figures, like reference numerals refer to the same or similar elements.
Fig. 1 is a block diagram of a lease management system for an ultrasonic system according to an embodiment; and
Fig. 2 is a flowchart of a lease management method for an ultrasonic system according to another embodiment.

### DETAILED DESCRIPTION

In the following detailed description, numerous specific details are set forth by way of example in order to provide a thorough understanding of the relevant teachings. However, the scope of the present disclosure is not limited to the specific details.

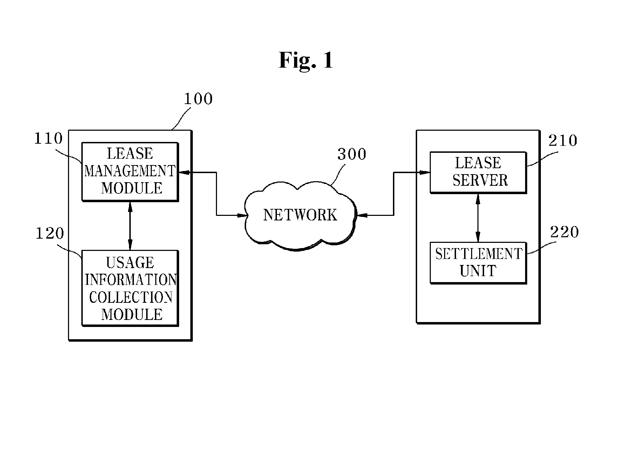

Referring to Fig. 1, a lease management system for an ultrasonic system according to the exemplary embodiment includes a usage information collection unit 120, a lease management unit 110, a lease server 210, and a settlement unit 220. The usage information collection unit 120 collects usage information regarding a usage of the ultrasonic system. The lease management unit 110 determines whether the usage information provided from the usage information collection module 120 exceeds a predetermined allowable usage set by a prepaid lease balance of a user. The lease management unit 110 stops the operation of the ultrasonic system 100 while displaying a settlement-request screen on a display unit (not shown) of the ultrasonic system 100, if the usage information exceeds the allowable usage. The lease server 210 remotely manages a lease of the ultrasonic system 100 through communication with the lease management unit 110 via a network 300. The settlement unit 220 makes payment of lease costs through communication with the lease server 210.

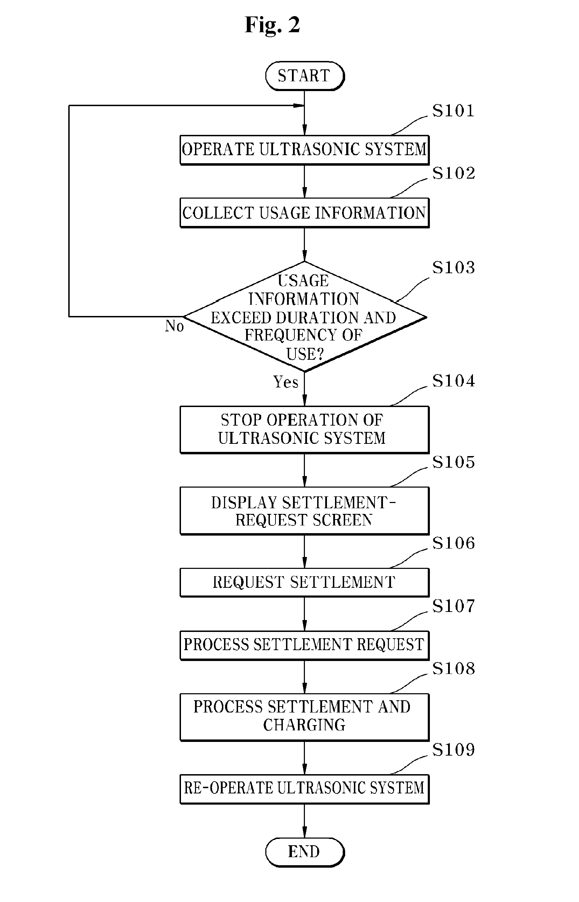

Operation of the lease management system according to the embodiment will be described in detail with reference to Figs. 1 and 2.

First, a user operates and uses an ultrasonic system within a preset allowable usage (allowable use range) or an allowable usage set by a prepaid lease balance of the user, in S101.

During operation of the ultrasonic system 100, the usage information collection unit 120 collects usage information regarding a usage of the ultrasonic system 100 in S102, and provides the usage information to the lease management unit 110.

Here, the usage information collected by the usage information collection module unit 120 includes at least one of duration and frequency of use of the ultrasonic system. Here, the duration of use and the frequency of use of the ultrasonic system may be linked with use of at least one of a main body, a probe, other optional or peripheral devices of the ultrasonic system, and combinations thereof.

Next, the lease management unit 110 determines whether amount of user's usage in the usage information exceeds an allowable usage set by a prepaid lease balance of the user in S103. Here, if the amount of user's usage in the usage information is within the predetermined allowable usage, the process proceeds to step S101 so that the ultrasonic system can be used continuously. On the contrary, if the amount of user's usage in the usage information exceeds the predetermined allowable usage, the lease management unit 110 controls the ultrasonic system 100 to stop operation in S104 and to display a settlement-request screen on a display unit (not shown) of the ultrasonic system 100 in S105.

Subsequently, if a user inputs settlement information to the settlement-request screen, the lease management unit 110 requests the lease server 210, via a network 300, to process a settlement according to the settlement information input by the user in S106. Then, the lease server 210 processes the settlement request provided from the lease management unit 110 and requests the settlement unit 220 to process the settlement in S107.

Next, the settlement unit 220 carries out a settlement process and a charging process according to the settlement request in S108. Specifically, according to the information for settlement of lease costs, which is input through the settlement-request screen by a user, the settlement unit 220 processes the settlement and charges a corresponding amount of money in S108, and provides a settlement process result to the lease server 210.

Then, the leaser server 210 provides information on the settlement process result including settlement and charged money to the lease management module 110, which in turn updates the allowable usage (time, frequency, etc.) of the ultrasonic system 100 in response to the settlement process result, and renders the ultrasonic system operable (i.e. mode update into operable mode). Thus, a user can re-operate the ultrasonic system 100 according to the updated condition in S109.

In one embodiment, instructions for performing the above method of managing lease of an ultrasonic system may be recorded in a computer readable medium using computer-readable instructions. The computer readable medium may include any type of record media, which can be read by a computer system. The computer readable medium may include read only memory (ROM), random access memory (RAM), CD-ROM, magnetic tape, floppy disk, optical-data recording apparatus and the like. The computer readable medium comprises instructions that, when executed by a processor performs collecting usage information regarding a usage of an ultrasonic system by a usage information collection module of the ultrasonic system during operation of the ultrasonic system, determining, by a lease management module of the ultrasonic system, whether the usage information exceeds a predetermined allowable usage according to a lease cost prepaid by a user, and stopping the operation of the ultrasonic system while displaying a settlement-request screen on a display unit of the ultrasonic system, if the usage information exceeds the predetermined allowable usage.

As set forth above, with the system for leasing an ultrasonic system, a user can conveniently use the ultrasonic system without an excessive initial cost burden and a provider can efficiently lease out the ultrasonic system with automatic update of an operation condition through electronic settlement and billing.

While the foregoing has described what are considered to be the best mode and/or other examples, it is understood that various modifications may be made therein and that the subject matter disclosed herein may be implemented in various forms and examples, and that the teachings may be applied in numerous applications, only some of which have been described herein. It is intended by the following claims to claim any and all applications, modifications and variations that fall within the true scope of the present teachings.

## Claims

1. A lease management system for an ultrasonic system, comprising:
a usage information collection unit to collect usage information regarding a usage of the ultrasonic system;
a lease management unit to determine whether the usage information provided from the usage information collection unit exceeds a predetermined allowable usage a prepaid lease balance of a user, and to stop operation of the ultrasonic system while displaying a settlement-request screen on a display unit of the ultrasonic system, if the usage information exceeds the allowable usage; and
a lease server remotely managing a lease of the ultrasonic system through communication with the lease management module via a network.

2. The system of claim 1, further comprising:
a settlement unit communicating with the lease server to carry out a settlement process,
wherein the lease management unit requests the lease server to process a settlement according to settlement information input through the settlement-request screen by a user,
the lease server requests the settlement unit to process the settlement, and
the settlement unit carries out a settlement process and a charging process according to the settlement request.

3. The system of claim 2, wherein the usage information comprises at least one of duration and frequency of use of the ultrasonic system.

4. The system of claim 3, wherein the duration and frequency of use of the ultrasonic system are linked with use of at least one of a main body, a probe, and an optional or peripheral device of the ultrasonic system.

5. A lease management method for an ultrasonic system, comprising:
collecting usage information regarding a usage of an ultrasonic system by a usage information collection unit during operation of the ultrasonic system;
determining, by a lease management unit , whether the usage information exceeds a predetermined allowable usage a prepaid lease balance of a user; and
stopping the operation of the ultrasonic system while displaying a settlement-request screen on a display unit if the usage information exceeds the predetermined allowable usage.

6. The method of claim 5, further comprising:
receiving settlement information from a user through the settlement-request screen;
requesting, by the lease management unit, a lease server to process a settlement via a network according to the settlement information input by the user;
requesting, by the lease server, a settlement unit to process the settlement request; and
carrying out, by the settlement unit, a settlement process and a charging process according to the settlement request.

7. The method of claim 6, further comprising:
providing a settlement process result from the settlement unit to the lease server;
providing information about the settlement process result from the lease server to the lease management module; and
updating, by the lease management module, the ultrasonic system into an operable mode in response to the information on the settlement process result.

8. The method of any one of claims 5 to 7, wherein the usage information includes at least one of duration and frequency of use of the ultrasonic system.

9. The method of claim 8, wherein the duration and frequency of use of the ultrasonic system are linked with use of at least one of a main body, a probe, and an optional or peripheral device of the ultrasonic system.
